Europäisches Patentamt

**European Patent Office**

**Office européen des brevets**

(19)

(11) Veröffentlichungsnummer: **0 150 760**
A2

(12)
# EUROPÄISCHE PATENTANMELDUNG

(21) Anmeldenummer: **85100363.2**

(51) Int. Cl.⁴: **A 61 K 37/02**

(22) Anmeldetag: **16.01.85**

---

(30) Priorität: **25.01.84 DE 3402466**

(43) Veröffentlichungstag der Anmeldung: **07.08.85**
**Patentblatt 85/32**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **von Gablenz, Elisabeth, Dr., Drei Linden Strasse 55, D-6232 Bad Sonden am Taunus (DE)**
Erfinder: **Eyndels, Christian, avenue de la Fauconnerie 117, B-1170 Brüssel (BE)**
Erfinder: **Hendrickx, Philippe, Dr., rue Chêne au Corbeau 37, B-1338 Lasne (BE)**

---

(54) **Sekretin und diese enthaltende Mittel zur Prophylaxe des Aspirations-Syndroms.**

(57) Die Erfindung betrifft die Verwendung von Sekretin und dieses enthaltende Mittel bei der Prophylaxe des Aspirations-Syndroms.

**EP 0 150 760 A2**

HOECHST AKTIENGESELLSCHAFT    HOE 84/F 017    Dr.WS/sch

Sekretin und dieses enthaltende Mittel zur Prophylaxe des
Aspirations-Syndroms

Das Aspirations-Syndrom, auch als Mendelson's Syndrom bekannt, tritt bei Narkose bzw. Verminderung der Reflexe
(zum Beispiel starke Sedation des Patienten) auf, wenn
Magensäure von einem pH-Wert kleiner als 2,5 in die Lunge
gelangt (Amer. J. of Obstetrics and Gynecology 52 (1946)
191-205). Die Symptome, die beobachtet werden, sind Zyanose, Dyspnöe, asthmaähnliches Keuchen und Rasseln über der
Brust, Tachykardie, Abfall des Blutdrucks, pulmonale
Ödeme und Herzversagen (Klinisches Wörterbuch mit klinischen Syndromen, 251. Auflage, Walter de Gruyter, Berlin-
New York (1972), Seite 756). Die Patienten sind gewöhnlich
in einem sehr kritischen Allgemeinzustand mit meist
infauster Prognose. Bei Kaiserschnitt ist das Aspirations-
Syndrom die häufigste narkosebedingte Todesursache
(Anesthesiology 51 (1979) 375-377).

Es ist bekannt, den pH-Wert des Magensaftes mit $H_2$-Rezep-
torenblockern zu erhöhen. Diese Erhöhung tritt jedoch
erst 45-90 Minuten nach der Applikation dieser Mittel ein
(Informationsbroschüre der Fa. Cascan GmbH über Sostril[R]
(1983) Seiten 70-72).

Für Notfalloperationen ist diese Zeit jedoch zu lang.

Es wurde nun gefunden, daß nach einer Bolus-Injektion von
Sekretin der pH-Wert des Magensaftes innerhalb weniger
Minuten schnell pH 2,5 überschreitet und nach 10 - 15 Minuten pH 6 - 8 erreicht. Sekretin eignet sich damit gut
zur Prophylaxe des Aspirations-Syndroms, insbesondere bei
Notfalloperationen.

Die Erfindung betrifft daher die Verwendung von Sekretin
zur Prophylaxe des Aspirations-Syndroms.

0150760

Sekretin ist ein Heptacosipeptidamid (Eur. J. Biochem. 15 (1970) 513), das im Duodenum gebildet wird und neben anderen Eigenschaften vor allem die Hydrogencarbonatproduktion der Bauchspeicheldrüse stimuliert und die Wirkung von Gastrin hemmt. Es wird bisher zur Diagnose und zur Therapie von Krankheiten des Verdauungstraktes verwendet. Die bei der Diagnostik der exokrinen Pankreasfunktion empfohlene i.v.-Dosis beträgt 1 klinische Einheit (CU) pro kg Körpergewicht (Rote Liste 1983, 34 019). Blutungen im Gastroduodenalbereich werden mit Sekretininfusionen behandelt. Man gibt etwa 0,5 CU pro kg Körpergewicht innerhalb einer Stunde. 1 mg Sekretin entspricht 4000 - 5000 CU (am Hund bestimmt).

Ein Verfahren zur Herstellung und Reinigung von Sekretin ist aus US-Patent 4 098 779 bekannt. In den vorstehenden und nachfolgenden Ausführungen schließt der Begriff Sekretin die physiologisch verträglichen Salze mit ein. Erfindungsgemäß wird sowohl natürliches als auch synthetisches Sekretin verwendet.

Gemäß der vorliegenden Erfindung werden 0,1 - 0,9, vorzugsweise 0,3 - 0,7, insbesondere 0,4 - 0,6 CU Sekretin pro kg Körpergewicht parenteral, vorzugsweise als i.v.-Bolus-Injektion verabreicht.

Die Erfindung betrifft auch ein pharmazeutisches Mittel aus einem pharmazeutisch unbedenklichen Träger und dem Wirkstoff Sekretin zur Anwendung bei der Prophylaxe des Aspirations-Syndroms.

Sekretin ist in Lösung nicht stabil (Life Sci. 29 (1981) 885-94). Sekretin wird deshalb in gefriergetrockneter Form gelagert und erst kurz vor Gebrauch gelöst. Da Sekretin häufig in sehr geringen Mengen verabreicht wird, ist es zweckmäßig, der Sekretinlösung vor der Lyophilisierung eine Trägersubstanz, vorzugsweise eine Aminosäure (vgl.

DE-OS 23 23 187), zuzusetzen, um die Gefahr einer Verstaubung zu vermeiden. Besonders bevorzugt ist Glycin.

Diese Zubereitung wird mit Salzsäure auf einen pH-Wert von 3 - 7, vorzugsweise auf etwa pH 4, eingestellt. Um die Adsorption des Sekretins an den Glas- und Kunststoffwandungen zu verhindern, gibt man natürliche Proteine, wie z.B. Human-Albumin oder Human-Globin, oder ein über Diisocyanate vernetztes Gelatinepartialhydrolysat (vgl. DBP 1 118 792 und 1 155 134) wie Haemaccel$^{(R)}$ zu. Die Adsorption an Glas- und Kunststoffmaterial läßt sich auch durch Zusatz von Detergentien, wie z.B. Lecithin, Copolymeren aus Ethylenoxid und Propylenoxid, Hydroxypropylcellulose, Methylcellulose, Polyoxyethylen und Polyethylenglykol verhindern (vgl. EP-A-95 751).

Da die Sekretinzubereitungen injiziert werden, ist es zweckmäßig, vor Applikation das Lyophilisat in Aqua pro injectione, das einen physiologisch verträglichen Puffer und ggf. ein übliches Isotoniemittel enthält, zu lösen.

Beispiel 1:
40 CU-Ampulle
Eine auf ca. 5°C gekühlte Lösung von 20 g Glycin in 400 ml Wasser wird mit 1N HCl auf pH 4 eingestellt. Dazu gibt man 2,5 g mit Diisocyanaten vernetztes Gelatinepartialhydrolysat (Haemaccel$^{(R)}$) in Form einer ca. 10%igen Lösung. In diese Trägerlösung werden 40 000 CU Sekretinhydrochlorid gelöst. Mit Wasser wird auf 500 ml aufgefüllt. Die Lösung wird durch ein geeignetes Filter steril filtriert und anschließend zu je 0,5 ml in Ampullen abgefüllt und gefriergetrocknet.

Beispiel 2:

Randomisierte Doppelblindstudie zur Beurteilung der Sicherheit und Wirksamkeit von Sekretin gegen Plazebo bei Notkaiserschnittpatientinnen (vgl. auch Tabelle 1)

Bei 20 Patientinnen mit Komplikationen während der Geburt mussten Notkaiserschnitte durchgeführt werden. 10-15 Minuten vor Induktion der Tiefnarkose erhielten sie entweder 0,5 CU/kg Körpergewicht Sekretin oder Plazebo als i.v. Bolus-Injektion. Sobald als möglich wurde eine nasogastrische Sonde eingeführt, um die Mageninhalte abzusaugen und deren pH-Wert zu bestimmen. Der gastrische pH-Wert wurde als Hauptwirksamkeitsparameter angewendet, da das Volumen von Magensekretionen kein sicherer Parameter dafür ist. Dieser kann größeren Abweichungen unterliegen, je nachdem wie kurz zuvor die Patientin etwas zu sich genommen hat.

Die Ergebnisse der Sekretingruppe waren wie folgt:

Eine Patientin (Pat. Nr. 14) wurde von der Auswertung der Wirksamkeit ausgeschlossen, da man ihr aufgrund ihres Gewichts von 150 kg die falsche Dosis gegeben hatte. Eine andere Patientin (Pat. Nr. 1) mußte von der Gesamtauswertung ausgeschlossen werden, da es sich als unmöglich erwies, Magensekretionen bei den zweiten und dritten Meßzeiten abzusaugen, und ihre Daten daher unvollständig waren. Innerhalb von 25 Minuten nach Injektion wurden pH-Werte über 6,0 bei 7 der 8 auswertbaren Fälle beobachtet. Der pH-Wert blieb unter 2,5 für Patientin Nr. 3. Diese Patientin hatte jedoch ein sehr kleines Absaugvolumen, so dass in diesem Falle die Gefahr eines Aspirationssyndroms ausgeschlossen werden kann.

Die Befunde für alle 10 Patientinnen der Plazebogruppe konnten ausgewertet werden. Bei drei Fällen war der gastrische pH-Wert grösser als 2,5. Bei drei anderen Fällen wurden mindestens 25 ml Magensaft mit einem pH-Wert unter 2,5 abgesaugt. Die Patientinnen wurden als aspirationssyndromgefährdet betrachtet, zumal das Absaugvolumen über 25 ml lag.

Da fast das Gesamtvolumen der Magensekretionen für die erste Messung abgesaugt wurde, ist es notwendig,

0150760

die Volumina der nachfolgenden Meßzeiten hinzuzurechnen, um die mögliche Gefährdung jeder Patientin zu bestimmen.

Zusammenfassend kann gesagt werden, dass keine (0/8) der Patientinnen in der Sekretingruppe, aber 6 (6/10) in der Plazebogruppe, aspirationssyndromgefährdet waren.

Tabelle 1: Wirksamkeitsparameter

| | Pat. Nr. | Zeit (Min.) nach Inj. | pH-Wert (titr.) | Magen-saft (ml) | Zeit (Min.) nach Inj. | pH-Wert (titr.) | Magen-saft (ml) | Summe Magen-saft (ml) | Gefahr* |
|---|---|---|---|---|---|---|---|---|---|
| Sekretin | 1" | 10 | 1,6 | 70 | - | - | - | - | - |
| | 2 | 15 | 1,4 | 10 | 20 | 7,0 | 50 | 64 | - |
| | 3 | 25 | 1,9 | 10 | 30 | 2,1 | 6 | 17 | - |
| | 8 | 15 | 6,2 | 45 | 20 | 7,4 | 17 | 65 | - |
| | 11 | 15 | 6,6 | 70 | 20 | 6,7 | 70 | 165 | - |
| | 12 | 20 | 6,6 | 120 | 25 | 7,2 | 40 | 161 | - |
| | 13 | 20 | 1,5 | 25 | 25 | 6,5 | 10 | 37 | - |
| | 14" | 17 | 1,8 | 37 | 22 | 1,5 | 7 | 46 | - |
| | 19 | 15 | 1,2 | 36 | 25 | 7,4 | 40 | 91 | - |
| | 20 | 15 | 6,7 | 60 | 20 | 8,2 | 8 | 69 | - |
| Plazebo | 4 | 15 | 1,7 | 145 | 25 | 1,7 | 25 | 188 | + |
| | 5 | 28 | 1,7 | 20 | 33 | 1,5 | 10 | 32 | + |
| | 8 | 12 | 2,7 | 6 | 17 | 2,2 | 10 | 17 | - |
| | 7 | 19 | 1,9 | 25 | 24 | 2,9 | 12 | 39 | - |
| | 9 | 15 | 1,5 | 80 | 20 | 1,7 | 25 | 115 | + |
| | 10 | 12 | 3,4 | 20 | 22 | 6,5 | 10 | 45 | - |
| | 15 | 10 | 4,8 | 35 | 20 | 7,6 | 1 | 37 | - |
| | 16 | 15 | 2,5 | 25 | 20 | 2,2 | 2 | 27 | + |
| | 17 | 15 | 1,4 | 5 | 20 | 1,7 | 30 | 40 | + |
| | 18 | 12 | 1,7 | 12 | 17 | 1,6 | 22 | 36 | + |

* Diagnose des Mendelson's SyndrompH-Wert <2,5, Volumen >25 ml
" Patientinnen von der Auswertung der Wirksamkeit ausgeschlossen

Patentansprüche:

1. Sekretin zur Anwendung bei der Prophylaxe des Aspirations-Syndroms.

2. Verwendung von Sekretin zur Prophylaxe des Aspirations-Syndroms.

3. Pharmazeutisches Mittel aus einem pharmazeutisch unbedenklichen Träger und dem Wirkstoff Sekretin zur Anwendung bei der Prophylaxe des Aspirations-Sysdroms.

4. Mittel nach Anspruch 3, dadurch gekennzeichnet, daß es einen pH-Wert zwischen 3 und 7 aufweist.

5. Mittel nach Anspruch 3 oder 4, dadurch gekennzeichnet, daß es ein mit Diisocyanaten vernetztes Gelatinepartialhydrolysat, Human-Albumin oder Human-Globin enthält.

6. Mittel nach einem der Ansprüche 3 bis 5, dadurch gekennzeichnet, daß es abgepuffertes Glycin enthält.